# EUROPEAN PATENT APPLICATION

(11) **EP 3 991 790 A1**
(43) Date of publication of application: **04.05.2022**
(21) Application number: 20832118.2
(22) Date of filing: 16.06.2020
(51) Int. Cl.: A61P 3/00, A61P 3/04, A61P 9/00, A61P 9/04, A61P 9/10, A61P 13/12, A61P 21/00, A61P 21/06, A61P 25/00, A61P 25/08, A61P 25/14, A61P 25/16, A61P 25/28, A61P 27/02, A61P 35/00, A61P 43/00, A23L 33/175, A61K 31/197, A61K 31/198

(54) **MITOCHONDRIAL FUNCTION ACTIVATOR**

(30) Priority: 28.06.2019 JP 2019121874
(71) Applicant: Yuki Gosei Kogyo Co., Ltd., Chuo-ku, Tokyo 103-0013 (JP); Kyushu University, National University Corporation, Nishi-ku Fukuoka-shi Fukuoka 819-0395 (JP)
(72) Inventor: NODA, Tetsuji, Tokyo 174-0043 (JP); ONAGA, Tomotsune, Tokyo 174-0043 (JP); KATAKURA, Yoshinori, Fukuoka-shi, Fukuoka 819-0395 (JP)
(74) Representative: Gille Hrabal Partnerschaftsgesellschaft mbB Patentanwälte
(86) International application number: PCT/JP2020/023600
(87) International publication number: WO 2020/262114

(57) **Abstract**

The object of the present invention is to provide a pharmaceutical agent which activate mitochondrial function, particularly, an inexpensive pharmaceutical agent.

The problem can be solved by a mitochondrial function activator of the present invention, comprising β-alanine or glycine, or a salt thereof.

SEQUENCE LISTING

<110>  YUKI GOSEI KOGYO CO., LTD.
       KYUSYU UNIVERSITY, NATIONAL UNIVERSITY CORPORATION

<120>  Mitochondrial function activating agent

<130>  YGK-046

<150>  JP 2019-121874
<151>  2019-06-28

<160>  8

<170>  PatentIn version 3.5

<210>  1
<211>  19
<212>  DNA
<213>  Artificial Sequence

<220>
<223>  beta-actin  forward primer

<400>  1
ggccaggtca tcactattg                                                    19


<210>  2
<211>  21
<212>  DNA
<213>  Artificial Sequence

<220>
<223>  beta-actin  reverse primer

<400>  2
gaggtcttta cggatgtcaa c                                                 21


<210>  3
<211>  20
<212>  DNA
<213>  Artificial Sequence

<220>
<223>  Pgc-1alfa  forward primer

<400>  3
aatgcagcgg tcttagcact                                                   20


<210>  4
<211>  20
<212>  DNA
<213>  Artificial Sequence

<220>
<223>  Pgc-1alfa  reverse primer

<400>  4
tgttgacaaa tgctcttcgc                                                   20

<210>  5
<211>  17
<212>  DNA
<213>  Artificial Sequence

<220>
<223>  Acaa1b  forward primer

<400>  5
tgagcggttt ggcgttt                                                      17


<210>  6
<211>  22
<212>  DNA
<213>  Artificial Sequence

<220>
<223>  Acaa1b  reverse primer

<400>  6
cttgtcaccc ttgtcattca gg                                                22


<210>  7
<211>  21
<212>  DNA
<213>  Artificial Sequence

<220>
<223>  Acaa2  forward primer

<400>  7
tgcccctcag ttcttgtctg t                                                 21


<210>  8
<211>  19
<212>  DNA
<213>  Artificial Sequence

<220>
<223>  Acaa2  reverse primer

<400>  8
aggtgtgcgg tgattctgg                                                    19

## Description

### TECHNICAL FIELD

The present invention relates to a mitochondrial function activator, a food composition for activating mitochondrial function, and a method for activating mitochondrial function. According to the present invention, the number of mitochondria in cells can be increased, and a mitochondrial activity can be enhanced.

### BACKGROUND ART

Mitochondria are eukaryotic organelles. It is thought that they are responsible for the production of ATP and play an important role in apoptosis. Mitochondria are abundant in cells of metabolically active tissues such as a liver, kidney, muscle, or brain. For example, fast muscle fibers of muscle have few mitochondria. However, slow muscle or myocardium fibers have many mitochondria, and the mitochondria produce ATP from glycogen via lactic acid.

Diseases caused by mitochondrial dysfunction include various diseases such as mitochondrial disease, neurodegenerative disease, immune neurological disease, cerebral ischemic disease, renal disease, muscle disease, or heart disease. It is expected that the symptoms will be improved by enhancing mitochondrial activity. In addition, even in healthy humans, athletic ability can be improved by activating the mitochondrial function of skeletal muscle.

### CITATION LIST

### PATENT LITERATURE

[Patent literature 1] JP 2015-97507 A
[Patent literature 2] JP 2014-162792 A

### SUMMARY OF INVENTION

### TECHNICAL PROBLEM

Carnosine or anserine (Patent literature 1) and β-citryl-L-glutamic acid (Patent literature 2) are known, as compounds that activate mitochondrial function. However, activation of mitochondrial function by these compounds is not sufficient. In addition, carnosine, anserine, and β-citryl-L-glutamic acid are expensive dipeptides, and an inexpensive method for activating mitochondria was desired.

Therefore, the object of the present invention is to provide a pharmaceutical agent which activates mitochondrial function, and particularly to provide an inexpensive pharmaceutical agent.

### SOLUTION TO PROBLEM

The present inventors have conducted intensive studies into a pharmaceutical agent which activates mitochondrial function, as a result, surprisingly found that β-alanine or glycine exhibits an excellent mitochondria function activation effect

The present invention is based on the above findings.

Accordingly, the present invention relates to:
[1] a mitochondrial function activator comprising β-alanine or glycine, or a salt thereof,
[2] the mitochondrial function activator of item [1], for improving fat burning, or aerobic exercise ability,
[3] the mitochondrial function activator of item [1], for preventing or treating a disease caused by mitochondrial dysfunction,
[4] the mitochondrial function activator of item [3], wherein the disease caused by mitochondrial dysfunction is a mitochondrial disease, neurodegenerative disease, immune neurological disease, cerebral ischemic disease, renal disease, muscle disease, or heart disease,
[5] the mitochondrial function activator of item [4], wherein the mitochondrial disease is a Leigh encephalopathy, stroke-like episodes syndrome (MELAS), chronic progressive external ophthalmoplegia (CPEO), Kearns-Sayre syndrome (KSS), myoclonus epilepsy associated with ragged-red fibers (MERRF), Pearson syndrome, Leber's hereditary optic neuropathy (LHON), mitochondrial encephalomyopathy, Barth syndrome, or lactic acidosis,
[6] the mitochondrial function activator of item [4], wherein the neurodegenerative disease is an amyotrophic lateral sclerosis (ALS), Parkinson's disease, Alzheimer's disease, Huntington's disease, Friedreich ataxia, multiple system atrophy, progressive supranuclear palsy, spinocerebellar degeneration, spinal muscular atrophy, spinal and bulbar muscular atrophy, or Charcot-Marie-Tooth disease,
[7] the mitochondrial function activator of item [4], wherein the immune neurological disease is a Guillain-Barre syndrome, multiple sclerosis, Miller Fisher syndrome, chronic inflammatory demyelinating polyneuropathy, or myasthenia gravis,
[8] the mitochondrial function activator of item [4], wherein the cerebral ischemic disease is a cerebral infraction,
[9] the mitochondrial function activator of item [4], wherein the renal disease is a renal failure, amyloid kidney, membranous nephropathy, focal glomerular sclerosis, IgA nephropathy, acute tubular necrosis, nephrotic syndrome, diabetic nephropathy, gouty kidney, renal edema, kidney tumor, kidney ischemia disorder, renal ischemia-reperfusion disorder, or cystic kidney,
[10] the mitochondrial function activator of item [4], wherein the muscle disease is a progressive muscular dystrophy, myotonic dystrophy, congenital myopathy, metabolic myopathy, distal myopathy, inflammatory myopathy, age-related muscle atrophy (sarcopenia), or disuse muscular atrophy,
[11] the mitochondrial function activator of item [4], wherein the heart disease is a myocardial infarction, cardiac failure, ischemic heart disease, or cardiac myopathy,
[12] a food composition for activating mitochondrial function, comprising β-alanine or glycine, or a salt thereof,
[13] a method for activating mitochondrial function, comprising a step of administering the mitochondrial function activator of item [1] to a subject,
[14] the method for activating mitochondrial function of item [13], for improving a fat burning or improving an aerobic exercise ability of the subject,
[15] an exercise training method for enhancing ability in aerobic exercise or increasing slow muscle, comprising a step of ingesting the mitochondrial function activator of item [1] to a subject,
[16] a dietary method for enhancing ability in aerobic exercise or increasing slow muscle, comprising a step of ingesting the mitochondrial function activator of item [1] to a subject,
[17] A Pgc-1α gene activator comprising β-alanine or glycine, or a salt thereof,
[18] an Acaalb gene activator comprising β-alanine or glycine, or a salt thereof, and
[19] an Acaa2 gene activator comprising β-alanine or glycine, or a salt thereof.

Further, the present specification discloses:
[20] a method for preventing or treating a disease caused by mitochondrial dysfunction, comprising a step of administering a therapeutic effective amount of β-alanine or glycine, or a salt thereof, and
[21] the method for preventing or treating a disease caused by mitochondrial dysfunction of the item [20], wherein the disease caused by mitochondrial dysfunction is a mitochondrial disease, neurodegenerative disease, immune neurological disease, cerebral ischemic disease, renal disease, muscle disease, or heart disease.

### ADVANTAGEOUS EFFECTS OF INVENTION

According to the mitochondrial function activator and the food composition for activating mitochondrial function of the present invention, the number of mitochondria in cells can be increased, and a mitochondrial activity can be enhanced. Further, β-alanine and glycine are easily available, and inexpensive mitochondrial function activators and the like can be provided.

### BRIEF DESCRIPTION OF THE DRAWINGS

Fig. 1 is a graph showing the increase in the number of mitochondria when undifferentiated mouse skeletal muscle-derived myoblast line, C2C12 cells were treated with β-alanine for 2 hours and 48 hours.
Fig. 2 is a graph showing the increase in the number of activated mitochondria when undifferentiated mouse skeletal muscle-derived myoblast line, C2C12 cells were treated with β-alanine for 2 hours and 48 hours.
Fig. 3 is a graph showing the increase in the number of mitochondria when undifferentiated mouse skeletal muscle-derived myoblast line, C2C12 cells were treated with glycine for 2 hours and 48 hours.
Fig. 4 is a graph showing the increase in the number of activated mitochondria when undifferentiated mouse skeletal muscle-derived myoblast line, C2C12 cells were treated with glycine for 2 hours and 48 hours.
Fig. 5 is a graph showing the increase in the number of mitochondria when mouse skeletal muscle-derived myoblast line, C2C12 cells, differentiated into myotube cells were treated with glycine for 2 hours and 48 hours.
Fig. 6 is a graph showing the increase in the number of activated mitochondria when mouse skeletal muscle-derived myoblast line, C2C12 cells, differentiated into myotube cells were treated with glycine for 2 hours and 48 hours.
Fig. 7 is a graph showing the expression of Pgc-1α gene when mouse skeletal muscle-derived myoblast line, C2C12 cells, differentiated into myotube cells were treated with β-alanine or glycine.
Fig. 8 is a confocal laser scanning micrograph when undifferentiated mouse skeletal muscle-derived myoblast line, C2C12 cells were treated with β-alanine for 48 hours.
Fig. 9 is a graph of measurement of fluorescence of Mito Tracker Red, when undifferentiated mouse skeletal muscle-derived myoblast line, C2C12 cells were treated with β-alanine for 72 hours.
Fig. 10 is a graph showing the expression of Acaalb gene or Acaa2 gene when mouse skeletal muscle-derived myoblast line, C2C12 cells, differentiated into myotube cells were treated with β-alanine.

### DESCRIPTION OF EMBODIMENTS

### [1] Mitochondrial function activator

The mitochondrial function activator of the present invention comprises β-alanine or glycine, or a salt thereof. Further, the mitochondrial function activator of the present invention may comprise another amino acid having a carboxyl group and an amino group, or a salt thereof, or the like. The mitochondrial function activator may comprise β-alanine or glycine, or either of salt thereof, or another amino acid having a carboxyl group and an amino group, or either of salt thereof, or the like, alone or a combination of two or more.

β-alanine is a compound of the following formula [1]:
[Chem. 1] 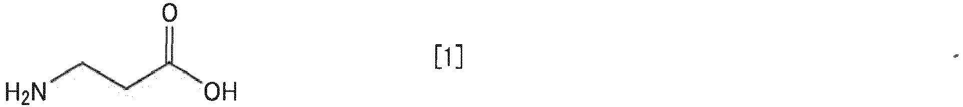 and is also referred to 3-aminopropanoic acid. As β-alanine comprised in the mitochondrial function activator of the present invention, extracts, concentrates, refined products, or the like from foods or natural products containing a relatively large amount of β-alanine can be used. Further, synthesized β-alanine may be used.
For example, β-alanine can be synthesized by β-alanine synthesis method from β-propiolactone (Ford, Org. Sys. Coll. Vol. 3, 34 (1955)). As another synthesis method, β-alanine can be synthesized from acrylonitrile and ammonia.

Glycine is a compound of the following formula [2].
[Chem. 2] 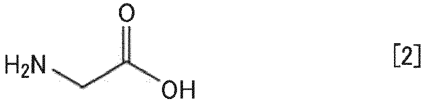 As glycine comprised in the mitochondrial function activator of the present invention, extracts, concentrates, refined products, or the like from foods or natural products containing a relatively large amount of glycine can be used. Further, synthesized glycine may be used. As a method for synthesizing glycine, there may be mentioned a Strecker method wherein aminoacetonitrile (glycinonitrile) is synthesized from formaldehyde, hydrogen cyanide, and ammonia, and it is hydrolyzed with sodium hydroxide or the like to produce a metal salt of glycine, and then it is neutralized with an acid such as sulfuric acid. Further, there may be mentioned a hydantoin method wherein glycolonitrile is synthesized from formaldehyde and hydrogen cyanide, and it is reacted with ammonia and carbon dioxide in the presence of water to produce hydantoin, and then it is hydrolyzed.

The salt of β-alanine or glycine is not limited, so long as it is a salt with an inorganic base or an organic base, or a salt with an acid, and is a salt acceptable for medicine or food. Specific examples of the salt with the inorganic base or the organic base include a salt with an inorganic base, an organic base, or a metallic alkoxide. They can be prepared by mixing β-alanine or glycine with an inorganic base, an organic base, or a metallic alkoxide.

As the inorganic bases that can form salts, there may be mentioned a hydroxide, carbonate, hydrogen carbonate, acetate, or hydride of alkali metals (such as lithium, sodium, potassium, or the like); a hydroxide, hydride, or the like of alkaline earth metals (such as magnesium, calcium, or barium). As the organic bases that can form salts, there may be mentioned dimethylamine, triethylamine, piperazine, pyrrolidine, piperidine, 2-phenylethylamine, benzylamine, ethanolamine, diethanolamine, pyridine, collagen, or the like. Further, as the metallic alkoxide, there may be mentioned sodium methoxide, potassium tert-butoxide, magnesium methoxide, or the like. The salt of β-alanine or glycine is preferably a sodium salt, potassium salt, calcium salt, or a combination thereof.

Specific examples of the salt with an acid include a salt with an inorganic acid or an organic acid. As the inorganic acid that can form a salt, there may be mentioned hydrochloric acid.

Further, as mitochondrial activators, other amino acids having a carboxyl group and an amino group can also be used, in addition to β-alanine or glycine in the present invention. Specifically, there may be mentioned amino acids that make up proteins such as valine, leucine, isoleucine, alanine, arginine, glutamine, lysine, aspartic acid, glutamic acid, proline, threonine, histidine, phenylalanine, tyrosine, tryptophan, asparagine, serine and the like; and further free amino acids in the living body such as phosphoserine, phosphoethanolamine, hydroxyproline, sarcosine, and α-aminoadipic acid citrullin, α-amino-n-butyric acid, β-aminoisobutyric acid, γ-aminobutyric acid, 3-methylhistidine, 1-methylhistidine, carnosine, anserine, hydroxylysine, ornithine, ethanolamine, carnitine, and the like, but it is not particularly limited thereto.

In addition to β-alanine or glycine of the present invention, a derivative of β-alanine or glycine can also be used. In the present specification, the derivative of β-alanine or glycine means a compound in which a hydrogen atom bonded to methylene carbon in the main chain is substituted. Examples of the substituent include an alkyl group having 1 to 3 carbon atoms.

### «Mitochondria»

Mitochondria are eukaryotic organelles, and have unique mitochondrial DNA. It is a place for oxygen respiration (aerobic respiration) and produces ATP. Further, it is thought that they play an important role in apoptosis.

Fast muscle fibers have few mitochondria, and thus lactic acid is easily produced by glycolysis. On the other hand, slow muscle and myocardium fibers have many mitochondria, and lactic acid is taken up therein and used as an energy source to produce ATP.

In addition, mitochondria play an important role in apoptosis. Stress such as DNA damage changes the membrane potential of mitochondria via the p53 which is apoptosis-inducing molecule, or the Bcl-2 family protein which regulates apoptosis. As a result, cytochrome c leaks from mitochondria and apoptosis is induced.

### <<Improvement of aerobic exercise ability»

According to the mitochondrial function activator of the present invention, the aerobic exercise ability is improved. In addition, according to the mitochondrial function activator of the present invention, the fat burning can be improved. As the aerobic exercise, there may be mentioned a running, walking, jogging, cycling, cross-country skiing, aerobics dance, STEP exercise, swimming, or water aerobics.

The mitochondrial function activator of the present invention can be used for enhancing the ability in the aerobic exercise. Mitochondria play a role in producing energy mainly using oxygen in cells. Aerobic energy metabolism takes place in the mitochondria. That is, the ability of aerobic exercise can be enhanced by activating mitochondria. In addition, mitochondrial activation is considered to have the same effect as endurance training, and thus the mitochondrial function activator of the present invention is useful for enhancing endurance in aerobic exercise. Further, it can be used for endurance-requiring sports in aerobic exercise, such as long-distance running, swimming, racewalking, triathlon, road racing, and soccer. Therefore, the mitochondrial function activator of the present invention can be used as an exercise training method related to aerobic exercise, by administering the mitochondrial function activator of the present invention to a subject. The exercise training method is not medical practice.

Furthermore, the effect of fat burning can be obtained by enhancing the aerobic exercise ability through the mitochondrial activation.

### «Disease caused by mitochondrial dysfunction»

According to the mitochondrial function activator of the present invention, the disease caused by mitochondrial dysfunction can be prevented or treated. The diseases caused by mitochondrial dysfunction is not particularly limited, but there may be mentioned mitochondrial disease, neurodegenerative disease, immune neurological disease, cerebral ischemic disease, renal disease, muscle disease, or heart disease. The mitochondrial disease is a disease in which sufficient aerobic energy production cannot be achieved due to mutations in mitochondria. Further, a mitochondrial dysfunction inhibits the activity of tissues with high energy demand, and thus it is known to cause the various diseases other than mitochondrial disease, such as neurodegenerative disease, immune neurological disease, cerebral ischemic disease, renal disease, muscle diseases, or heart disease. Therefore, the mitochondrial function activator of the present invention can increase the number of mitochondria, promote tissue regeneration, and treat these diseases.

The mitochondrial disease is not limited, but there may be mentioned a Leigh encephalopathy, stroke-like episodes syndrome (MELAS), chronic progressive external ophthalmoplegia (CPEO), Kearns-Sayre syndrome (KSS), myoclonus epilepsy associated with ragged-red fibers (MERRF), Pearson syndrome, Leber's hereditary optic neuropathy (LHON), mitochondrial encephalomyopathy, Barth syndrome, lactic acidosis, or the like.

The neurodegenerative disease is not limited, but there may be mentioned an amyotrophic lateral sclerosis (ALS), Parkinson's disease, Alzheimer's disease, Huntington's disease, Friedreich ataxia, multiple system atrophy, progressive supranuclear palsy, spinocerebellar degeneration, spinal muscular atrophy, spinal and bulbar muscular atrophy, Charcot-Marie-Tooth disease, or the like.

The immune neurological disease is not limited, but there may be mentioned a Guillain-Barre syndrome, multiple sclerosis, Miller Fisher syndrome, chronic inflammatory demyelinating polyneuropathy, myasthenia gravis or the like.

The cerebral ischemic disease is not limited, but there may be mentioned a cerebral infraction.

The renal disease is not limited, but there may be mentioned a renal failure, amyloid kidney, membranous nephropathy, focal glomerular sclerosis, IgA nephropathy, acute tubular necrosis, nephrotic syndrome, diabetic nephropathy, gouty kidney, renal edema, kidney tumor, kidney ischemia disorder, renal ischemia-reperfusion disorder, cystic kidney, or the like.

The muscle disease is not limited, but there may be mentioned a progressive muscular dystrophy, myotonic dystrophy, congenital myopathy, metabolic myopathy, distal myopathy, inflammatory myopathy, age-related muscle atrophy (sarcopenia), disuse muscular atrophy, or the like.

The heart disease is not limited, but there may be mentioned a myocardial infarction, cardiac failure, ischemic heart disease, cardiac myopathy, or the like.

The "activation of function" by the mitochondrial function activator of the present invention is not particularly limited as long as the mitochondrial function is improved, but includes an increase of the number of mitochondria, or an increase of the number of activated mitochondria.

In addition, the "activation of function" by the mitochondrial function activator includes an activation of gene which improve the mitochondrial function. For example, the activation of gene includes an increase of transcription of a gene involved in mitochondrial biosynthesis, Pgc-1α. Further, there may be mentioned an increase of transcription of acetyl-coenzyme A acyltransferase 2 (Acaa2) and acetyl-coenzyme A acyltransferase 1b (Acaalb) involved in the step of the fatty acid beta-oxidation spiral of mitochondria.

The formulation of the mitochondrial function activator of the present invention is not particularly limited. For example, there may be mentioned oral agents, such as powders, subtle granules, granules, tablets, capsules, suspensions, emulsions, sylups, extracts, or balls; or parenteral agents, such as injections, liquid for external use, ointments, suppositorys, creams for local administration, or eye-drops.

The above oral agent can be prepared in accordance with conventional methods, using excipients, such as gelatin, alginate sodium, starch, cornstarch, saccharose, lactose, glucose, mannitol, carboxymethyl-cellulose, dextrin, polyvinyl pyrrolidone, crystalline cellulose, soy lecithin, sucrose, fatty acid ester, talc, magnesium stearate, polyethylene glycol, magnesium silicate, silicic anhydride, or synthetic aluminum silicate; binders, disintegrators, surfactants, lubricants, flow accelerator, diluents, preservatives, colorants, flavors, correctives, stabilizers, humectants, antiseptics, antioxidant, or the like.

Examples of the parenteral agents include injections. In a preparation of the injections, an aqueous solvent such as normal saline solution or Ringer solution, nonaqueous solvents such as plant oil or fatty acid ester, isotonic agents such as glucose or sodium chloride, a solubility assisting agent, a stabilizing agent, an antiseptic agent, a suspending agent, or an emulsifying agent, may be optionally used, in addition to the active ingredient.

A dose of the mitochondrial function activator may be appropriately determined in accordance with, for example, age, sex, body weight, or degree of symptom of each patient, the type of each active ingredient, type of each disease, route of administration, or the like, and the determined dosage can be administered orally or parenterally. For example, in the case of an adult, the intake amount of the mitochondrial function activator of the present invention is preferably 0.01 to 100 mg / kg per day as β-alanine or glycine. The above administration method is an example, and other administration methods may be used. It is desirable that the administration method, dose, administration period, administration interval, and the like, of the mitochondrial function activator to humans are determined by a controlled clinical trial.

In addition, dosage form for administration of the mitochondrial function activator is not limited to a drug medicine. That is, it can be administered as a food and drink of various form, such as a functional food, a healthy food (including drink), or an animal food stuff.

As a method for preparing a mitochondrial function activator containing β-alanine or glycine, known pharmaceutical preparation methods can be used except that β-alanine or glycine is contained as an active ingredient.

The mitochondrial function activator of the present invention may contain other components. Examples of the other components include, for example, emulsifiers such as edible fats and oils, water, glycerin fatty acid ester, sucrose fatty acid ester, sorbitan fatty acid ester, propylene glycol fatty acid ester, glycerin organic acid fatty acid ester, polyglyceryl fatty acid ester, calcium stearoyl lactylate, sodium stearoyl lactate, polyoxyethylene sorbitan fatty acid ester; thickening stabilizers such as locust bean gum, carrageenan, alginic acids, pectin, xanthan gum, crystalline cellulose, carboxymethyl cellulose, methyl cellulose, agar, glucomannan, gelatin, starch, or chemical starch; salty taste agents such as salt, or potassium chloride; acidulants such as acetic acid, lactic acid, or gluconic acid; sugars or sugar alcohols; sweeteners such as stevia or aspartame; colorants such as beta-carotene, caramel, or red koji pigment; antioxidants such as tocopherol or tea extract; food materials or food additives such as flavoring agent; pH adjuster; food preservative, or shelf life improver. Further, the mitochondrial function activator may contain various vitamins, or functional materials such as coenzyme Q, plant sterol, or milk fat globule membrane. Among the mitochondrial function activators of the present invention, the total amount is preferably 80% by mass or less, more preferably 40% by mass or less, and further preferably 20% by mass or less.

The mitochondrial function activator of the present invention can be administered to humans. Further, the subject to be administered may be animals other than human, that is, there may be mentioned pets such as dog, cat, rabbit, hamster, guinea pig, and squirrel; domestic animals such as cow, horse, and pig; experimental animals such as mouse and rat; animals bred in zoos, or the like.

### [2] Method for activating mitochondrial function

The method for activating mitochondrial function of the present invention comprises a step of administering the mitochondrial function activator to a subject. That is to say, said β-alanine or glycine can be used in the method for activating mitochondrial function. The mitochondrial function can be activated by administering an effective amount of the mitochondrial function activator to humans or animals. Further, the method for activating mitochondrial function of the present invention may be carried out when the subject suffers from a disease caused by mitochondrial dysfunction. Furthermore, the method for activating mitochondrial function of the present invention may be carried out for improving a fat burning or improving an aerobic exercise ability of the subject

In addition, β-alanine or glycine can be used for a method for enhancing aerobic exercise ability, or for an exercise training method for increasing slow muscle, via the mitochondrial function activation.

Further, β-alanine or glycine can be used for a method for preventing or treating a disease caused by mitochondrial dysfunction, via the mitochondrial function activation. That is to say, the method for preventing or treating a disease caused by mitochondrial dysfunction of the present invention comprises a step of administering a therapeutic effective amount of β-alanine or glycine, or a salt thereof to a patient of disease caused by mitochondrial dysfunction. The disease caused by mitochondrial dysfunction includes a mitochondrial disease, neurodegenerative disease, immune neurological disease, cerebral ischemic disease, renal disease, muscle disease, heart disease, Alzheimer's disease, or sarcopenia.

### <<β-alanine or glycine for use of method for activating mitochondrial function»

Said β-alanine or glycine can be used for the method for activating mitochondrial function. That is to say, the present specification discloses β-alanine or glycine for use of method for activating mitochondrial function.

Further, β-alanine or glycine is used for the method for enhancing aerobic exercise ability, or for an exercise training method for increasing slow muscle. Furthermore, β-alanine or glycine is used for the method for preventing or treating a disease caused by mitochondrial dysfunction

### «Use of β-alanine or glycine for manufacturing mitochondrial function activator»

Said β-alanine or glycine can be used for manufacturing the mitochondrial function activator. That is to say, the present specification discloses a use of β-alanine or glycine for manufacturing the mitochondrial function activator.

Further, β-alanine or glycine can be used for manufacturing an agent for enhancing aerobic exercise ability, an agent for improving fat burning, or an agent for preventing or treating disease caused by mitochondrial dysfunction.

### [3] Food composition for activating mitochondrial function

The food composition for activating mitochondrial function of the present invention comprises β-alanine or glycine, or a salt thereof. The food composition for activating mitochondrial function may comprise β-alanine, glycine, or the salt thereof alone, or a combination of two or more thereof.

The food composition for activating mitochondrial function of the present invention may comprise the mitochondrial function activator, as long as it can be administered orally. That is to say, the food composition for activating mitochondrial function of the present invention is possible to use the conditions and the like described in the section of the mitochondrial function activator, and the same effect can be obtained, as long as it can be used orally.

The food in the food composition for activating mitochondrial function of the present invention is a food or drink, and includes a beverage. The food in the present invention is not particularly limited, for example, there may be mentioned seasonings such as a miso-paste, soy sauce, sauce for noodles, sauce, soup stock, pasta sauce, dressing, mayonnaise, tomato ketchup, Worcestershire sauce, sauce for pork cutlet, or sprinkle; instant cooked foods such as a soup base, curry roux, white sauce, rice with tea base, or soup base; soups such as miso soup, soup, consomme soup, or potage soup; processed livestock products such as grilled meat, ham, or sausage; processed marine products such as boiled fish paste, dried fish, salted fish guts, fish boiled in soy sauce, delicacy; processed vegetable products such as pickles; snacks such as potato chips, or rice cracker; bakery foods such as bread, sweet bread, or cookies; cooked foods such as boiled foods, fried foods, grilled foods, curry, stew, gratin, rice, porridge, or rice ball; noodles such as pasta, wheat noodle, or ramen; oil processed foods such as margarine, shortening, fat spread, or flavored fat spread; materials for confectionery and baking such as flower pastes, or bean paste; mixed flours such as bread mix powder, cake mix powder, or fried food mix powder; confectioneries such as chocolate, candy, jelly, ice cream, or gum; Japanese confectioneries such as steamed bun, or castella; beverages such as coffee, coffee milk, tea, milk tea, soy milk, nutritional drink, vegetable drink, vinegared drink, juice , cola, mineral water, or sports drink; alcoholic beverages such as beer, wine, cocktail, or sour; milk and dairy products such as bovine milk, yogurt, or cheese.

The food composition for activating mitochondrial function of the present invention can be prepared by using the known methods for manufacturing foods and drinks except for comprising β-alanine or glycine.

The food composition for activating mitochondrial function of the present invention exhibits the effect same as the mitochondrial function activator. Therefore, according to the food composition for activating mitochondrial function of the present invention, an aerobic exercise ability is enhanced. Further, a fat burning can be improved by the food composition for activating mitochondrial function of the present invention. Therefore, an effect of enhancing ability in aerobic exercise, increasing slow muscle, or the like, can be obtained by a dietary method wherein a subject ingests the food composition for activating mitochondrial function.

### [4] Exercise training method

The exercise training method of the present invention comprises a step of ingesting the mitochondrial function activator to a subject. The exercise training method of the present invention is effective for enhancing ability in aerobic exercise, or increasing slow muscle.

As the aerobic exercise, there may be mentioned a running, walking, jogging, cycling, cross-country skiing, aerobics dance, STEP exercise, swimming, or water aerobics.

The exercise training method of the present invention is effective for an endurance training. That is to say, it is useful for enhancing endurance in aerobic exercise, and it can be used for enhancing the ability in endurance-requiring sports of aerobic exercise, such as long-distance running, swimming, racewalking, triathlon, road racing, and soccer. These exercise training methods are not medical practices.

### [5] Dietary method

The dietary method of the present invention comprises a step of ingesting the mitochondrial function activator to a subject. The dietary method of the present invention is effective for enhancing ability in aerobic exercise or increasing slow muscle. The dietary method excludes medical practices.

### «Functions»

A mechanism by which β-alanine or glycine contained in the mitochondrial function activator or the like of the present invention, increases the number of mitochondria and activates mitochondrial function has not been completely elucidated. However, it is presumed that β-alanine or glycine acts directly on the activation of cells and mitochondria of the cells. In addition, the chemical structure of β-alanine or glycine is exactly the same except that the number of methylene carbon is two or one in the main chain. Thus, it is considered that they have a carboxyl group and an amino group, which is important for mitochondrial function activation. Therefore, many amino acids can be used as the active ingredient of the mitochondrial function activator of the present invention, but β-alanine, glycine, or γ-aminobutyric acid is preferable.

### EXAMPLES

The present invention will be specifically described with the following Examples, but these do not limit the scope of the present invention.

### <<Example 1>>

In this example, an activation of mitochondrial function by β-alanine was examined using undifferentiated mouse skeletal muscle-derived myoblast line, C2C12 cells.

C2C12 cells were subcultured at 37°C in the presence of 5% CO2, using Dulbecco's Modified Eagle Medium (DMEM) (Nissui, Tokyo, Japan) containing 10% inactivated FBS, penicillin (100,000 U/L; Meiji, Tokyo, Japan), streptomycin (100mg/L: Meiji), NaHCO₃ (2.0 g/L: Wako, Osaka, Japan), and L- glutamine (Wako).

As to β-alanine, 0.0089 g of β-alanine powder (Wako) was dissolved in 1 × PBS (1 mL) and sterilized by filtration using a 0.22 µM×13 mm filter (Merck Millipore, Billerica, MA, USA), to obtain a 100 mM of stock solution.

The number of mitochondria in C2C12 cells was measured as follows. C2C12 cells were seeded in 6-well plates at 4.0 × 10⁵ cells/well, and after 24 hours, β-alanine was added thereto at a concentration of 500 µM or 1 mM. In addition, 1 × PBS was added to as a control well. After 2 hours from the above addition, the medium was removed by suction, and 2 mL/well of Mito Tracker Green FM (Invitrogen) which is diluted to 200 nM in DMEM medium containing 10% FBS, was added. Then, the whole was incubated for 30 minutes at 37 °C in the presence of 5% CO₂ under shading. Then, the diluted solution was removed by suction, and 1 × PBS was added at 1 mL/well to wash the cells. 1 × PBS was removed by suction and trypsin was added at 500 µL/well. Then, trypsin was removed by suction and incubated at 37 °C in the presence of 5% CO₂ for 5 minutes under shading. After incubation, the cells were suspended to 1 mL of DMEM medium containing 10% FBS to be collected in a 1.5 mL tube. After passing this cell suspension through a nylon mesh (Kyoshin Riko, Tokyo, Japan), the cells were measured by a flow cytometer (EPICS XL System II-JK, Beckman Coulter, CA, USA; Cell Analyzer EC800 C5C, Sony, Tokyo, Japan). The analysis was performed at the FCM analysis software FlowJo (TOMY Digital Biology, Tokyo, Japan).

In addition, cells were seeded, and β-alanine was added thereto at a concentration of 100 µM or 1 mM. After 24 hours, β-alanine was secondary added, and the medium was aspirated after 24 hours of the second addition. Using the above cells treated with β-alanine for 48 hours as cells to which the diluted solution of Mito Tracker Green FM (Invitrogen) was added, the same procedure as the cells treated with β-alanine for 2 hours was repeated.

The number of activated mitochondria in C2C12 cells was measured as follows. C2C12 cells were seeded in 6-well plates at 4.0 × 10⁵ cells/well, and after 24 hours, β-alanine was added thereto at a concentration of 500 µM or 1 mM. In addition, 1 × PBS was added to as a control well. After 2 hours from the above addition, the medium was removed by suction, and 2 mL/well of Mito Tracker Red CMXRos (Invitrogen) (Invitrogen) which is diluted to 250 nM in DMEM medium containing 10% FBS, was added. Then, the whole was incubated for 30 minutes at 37 °C in the presence of 5% CO₂ under shading. Then, the diluted solution was removed by suction, and 1 × PBS was added at 1 mL/well to wash the cells. 1 × PBS was removed by suction and trypsin was added at 500 µL/well. Then, trypsin was removed by suction and incubated at 37 °C in the presence of 5% CO₂ for 5 minutes under shading. After incubation, the cells were suspended to 1 mL of DMEM medium containing 10% FBS to be collected in a 1.5 mL tube. After passing this cell suspension through a nylon mesh (Kyoshin Riko), the cells were measured by a flow cytometer (EPICS XL System II-JK, Beckman Coulter, CA, USA; Cell Analyzer EC800 C5C, Sony). The analysis was performed at the FCM analysis software FlowJo (TOMY Digital Biology, Tokyo, Japan).

In addition, cells were seeded, and β-alanine was added thereto at a concentration of 100 µM or 1 mM. After 24 hours, β-alanine was secondary added, and the medium was aspirated after 24 hours of the second addition. Using the above cells treated with β-alanine for 48 hours as cells to which the diluted solution of Mito Tracker Red CMXRos (Invitrogen) was added, the same procedure as the cells treated with β-alanine for 2 hours was repeated.

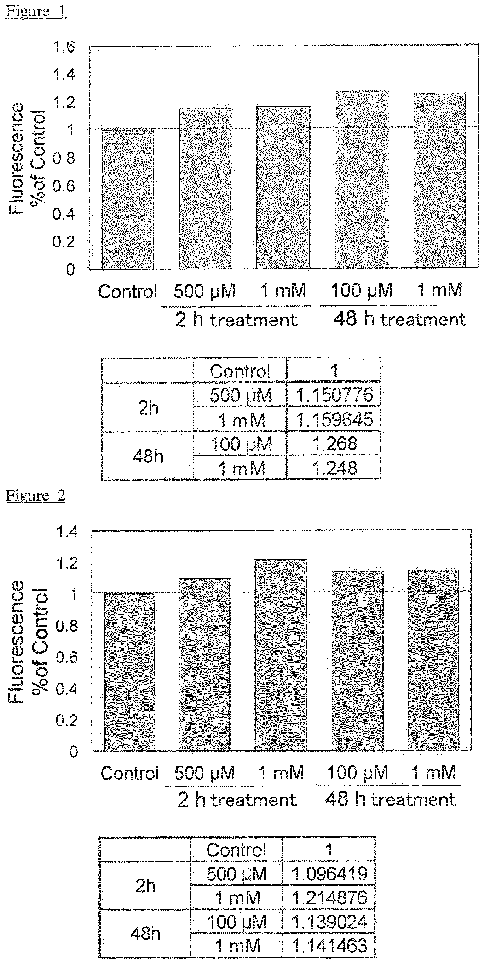

Mito Tracker Green FM (Invitrogen) is a fluorescent reagent that stains mitochondria independently of the membrane potential. Therefore, when a peak of the flow cytometer histogram shifts to the right by the addition of β-alanine, it indicates that the number of mitochondria is increased. As shown in Fig. 1, it was confirmed that the number of mitochondria increased in a concentration-dependent manner due to the addition of β-alanine to undifferentiated cells at both the treatment time of 2 hours and 48 hours.

On the other hand, Mito Tracker Red CMXRos (Invitrogen) is a fluorescent reagent that stains mitochondria in a membrane potential-dependent manner. Therefore, when a peak of the flow cytometer histogram shifts to the right by the addition of β-alanine, it indicates mitochondrial activity was enhanced. As shown in Fig. 2, mitochondrial activation was confirmed by the addition of β-alanine to undifferentiated cells at both the treatment time of 2 hours and 48 hours in a concentration-dependent manner.

### «Example 2»

In this example, an activation of mitochondrial function by glycine was examined using undifferentiated mouse skeletal muscle-derived myoblast line, C2C12 cells.

The procedure described in Example 1 was repeated, except that glycine was used instead of β-alanine.

A glycine solution was prepared as follows. 0.0075 g of glycine powder (Wako) was dissolved in 1×PBS (1 mL), and the solution was sterilized by filtration using a 0.22 µM × 13 mm filter (Merck Millipore) to obtain a 100 mM stock solution.

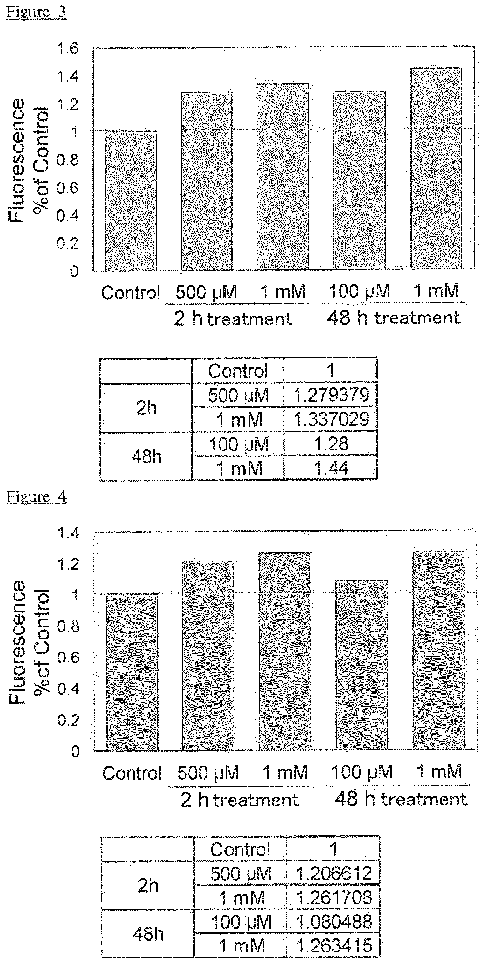

As shown in Fig. 3, it was confirmed that the number of mitochondria increased in a concentration-dependent manner due to the addition of glycine to undifferentiated cells at both the treatment time of 2 hours and 48 hours. As shown in Fig. 4, mitochondrial activation was confirmed by the addition of glycine to undifferentiated cells at both the treatment time of 2 hours and 48 hours in a concentration-dependent manner.

### «Example 3»

In this example, an activation of mitochondrial function by glycine was examined using mouse skeletal muscle-derived myoblast line, C2C12 cells which are differentiated into myotube cells. The procedure described in Example 2 was repeated, except that C2C12 cells which are differentiated into myotube cells was used instead of the undifferentiated C2C12 cells. The C2C12 cells were induced to differentiate into myotube cells as follows.

C2C12 cells were seeded, and cultured to about 90% confluency. Then, the cells were induced to differentiate by exchanging the medium with DMEM medium containing 2% Horse Serum (HS) (Invitrogen, Tokyo, Japan). Then, while exchanging the medium every 48 hours, cells were cultured for about 1 week at 37 °C in the presence of 5% CO₂ and the obtained cells were used as myotube cells in the experiment. Further, Mito Tracker Green FM (Invitrogen) and Mito Tracker Red CMXRos (Invitrogen) , were diluted in DMEM medium containing 2% HS, and the stained cells were collected in DMEM medium containing 2% HS.

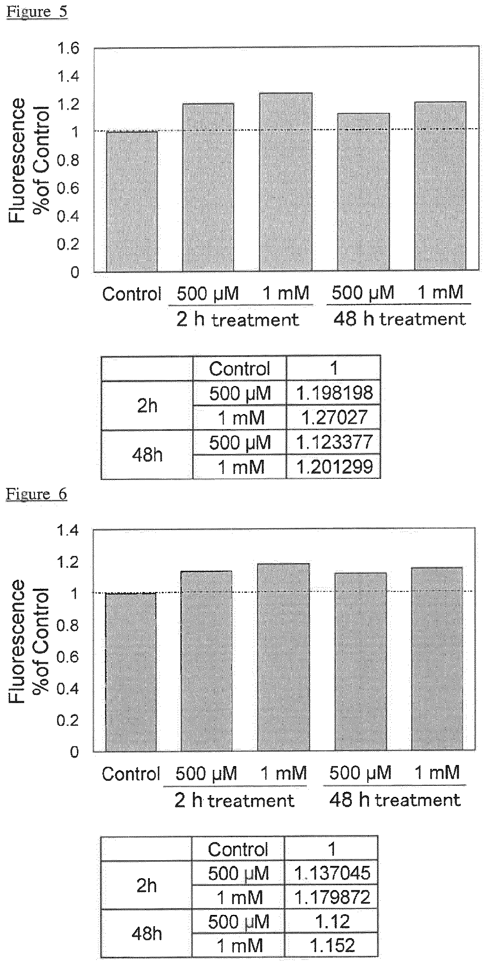

As shown in Fig. 5, it was confirmed that the number of mitochondria increased in a concentration-dependent manner due to the addition of glycine to differentiated cells at both the treatment time of 2 hours and 48 hours. In addition, as shown in Fig. 6. mitochondrial activation was confirmed by the addition of glycine to differentiated cells at both the treatment time of 2 hours and 48 hours, in a concentration-dependent manner.

### «Example 4»

In this example, a transcription of the Pgc-1α gene in mouse skeletal muscle-derived myoblast line C2C12 cells which were treated with β-alanine or glycine, was measured.

Total RNA in cells was prepared using the High Pure RNA Isolation Kit (Roche, Basel, Switzerland).

C2C12 cells were seeded in 6-well plates at 4.0×10⁵ cells/well, and cultured to about 90% confluency. Then, the cells were induced to differentiate by exchanging the medium with DMEM medium containing 2% Horse Serum (HS).
Then, while exchanging the medium every 48 hours, cells were cultured for about 1 week at 37 °C in the presence of 5% CO₂ and the obtained cells were used in the experiment. β-alanine and glycine were added to the differentiated cells, and 1 × PBS was added thereto as the control. After 24 hours of the addition, sample was secondary added, and the medium was aspirated after 24 hours of the second addition of sample. Then, 1 × PBS was added at 1 mL/well to wash the cells, and the cells were washed twice. Thereafter, 1 × PBS (200 µL/well) and Lysis/-binding buffer (400 µL/well) were added to spread to the whole dish, and then the whole volume was collected in a 1.5 mL tube. The collected sample was suspended in a vortex mixer for 60 seconds. The filter tube and the recovery tube were assembled. The sample solution was added to the filter tube, and was centrifuged at 4°C, at 10000 rpm for 15 seconds. The liquid discharged into the recovery tube was discarded, and the filter tube and recovery tube were reassembled. In a 1.5 mL tube, 90µL of DNase Incubation Buffer and 10µL of DNase I were mixed per sample. This mixture was added to the filter tube and incubated for 15 minutes at room temperature. After incubation, Wash buffer I (500µL) was added to the filter tube and centrifuged at 4°C, at 10000 rpm for 15 seconds. The liquid discharged into the recovery tube was discarded, and the filter tube and recovery tube were reassembled. Wash buffer II (500µL) was added to the filter tube, and centrifuged at 4°C, at 10000 rpm for 15 seconds. The liquid discharged into the recovery tube was discarded, and the filter tube and recovery tube were reassembled. Wash buffer II (200µL) was added to the filter tube, and centrifuged at 4°C, at 13000 rpm for 2 minutes. The filter tube was inserted into a new 1.5 mL tube, and Elution buffer (70µL) was added to the filter tube. It was allowed to stand at room temperature for 3 minutes, and then centrifuged at 4°C, at 10000 rpm for 1 minute. The eluate obtained by the above procedure was used as an RNA solution. The RNA concentration in the solution was calculated based on the absorbance at 260 nm using a NanoDrop 2000 / 2000c spectrophotometer (Thermo Scientific, Waltham, MA, USA) and the solution was used in subsequent experiments.

A cDNA was synthesized as follows. 0.5µL of Oligo (dT)₂₀ primer (10 pmoles/µL) was added to 1.0 µg of total RNA extracted from cells, and RNase Free water was added to a total volume of 13.5 µL. The mixture was suspended by tapping, and spun down. Then, it was heat-treated with Thermal Cycler PTC-200 (MJ Research, Waltham, MA, USA) at 65 ° C for 5 minutes, immediately transferred to ice and allowed to stand for 5 minutes. 4 µL of 5×RT Buffer (TOYOBO, Osaka, Japan), 2µL of 10mM dNTPs (GE Healthcare, Amersham Place, UK), and 0.5µL of reverse transcription enzyme, ReverTra Ace (100 units/µL) (TOYOBO) were added to 0.2 mL tube per 1 sample, and mixed by pipetting. 6.5 µL of this mixture was added to the tube that was allowed to stand for 5 minutes, and the whole was mixed by pipetting on ice and then spun down.
Then, a cDNA was synthesized by reacting at 42°C for 20 minutes and 99°C for 5 minutes, and used as a template for subsequent quantitative real-time PCR.

The quantitative RT-PCR was performed using the prepared cDNA as a template. 49µL of sterile water, 3.5µL of each of Primer Forward/Reverse (diluted to 10µM), 7.0µL of the template cDNA (diluted to 1/5 for Pgc-1α, and diluted to 1/50 for β-actin) were added to a PCR tube (0.2 mL) on ice. The mixture was spun down, and then 24.5µL of THUNDERBIRD SYBR qPCR Mix (TOYOBO) was added thereto and the whole was suspended well on ice. As a negative control, 17µL of sterile water, 0.5µL of each of Primer Forward/Reverse (diluted to10µM) were added to a PCR tube (0.2 mL). The mixture was spun down, and then 7µL of THUNDERBIRD SYBR qPCR Mix (TOYOBO) was added thereto and the whole was mixed well on ice. Thereafter, 25µL of the mixture was added to each of 3 wells of a 96-well PCR Plate (NIPPON Genetics, Tokyo, Japan), and quantitative RT-PCR was performed using the Thermal Cycle Dicer Real Time System (Takara, Shiga, Japan). The PCR reaction was performed by 1 cycle of 95°C for 30 seconds, and 40 cycles of 95°C for 5 seconds and 60°C for 60 seconds, and detected by FAM. The expression level of Pgc-1α was relatively quantified by comparing the Ct value of β-actin, which is one of the housekeeping genes, with the Ct value of Pgc-1α using the ΔΔCt method. The synthesis of the primers was outsourced to Sigma, and the sequences of the primers used is as follows.
β-actin forward:5'-GGCCAGGTCATCACTATTG-3' (SEQ ID No:1)
β-actin reverse:5'-GAGGTCTTTACGGATGTCAAC-3' (SEQ ID No:2)
Pgc-1α forward:5'-AATGCAGCGGTCTTAGCACT-3' (SEQ ID No:3)
Pgc-1α reverse:5'-TGTTGACAAATGCTCTTCGC-3' (SEQ ID No:4)

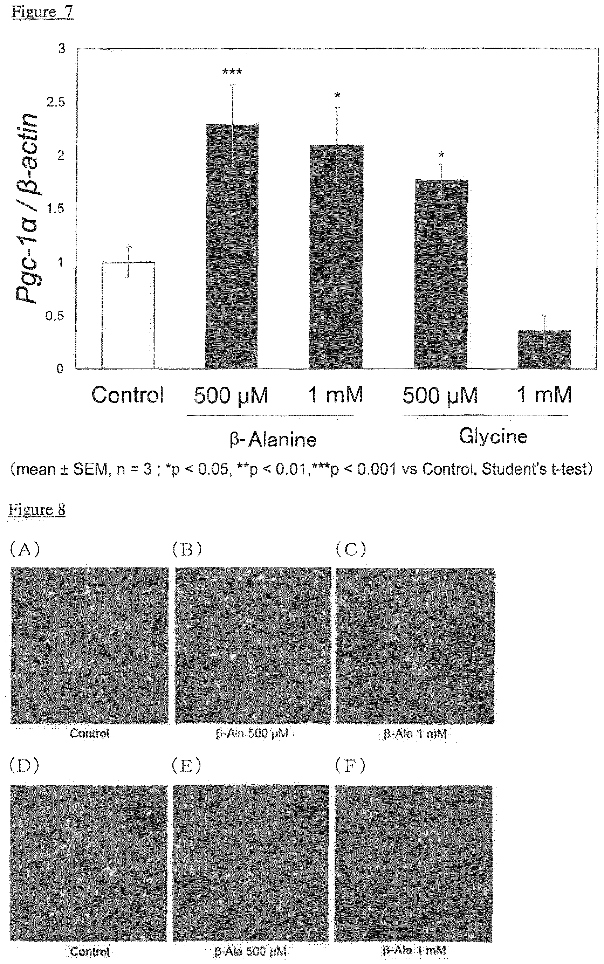

As shown in Figure 7, the treatment by β-alanine with 500µM and 1mM and the treatment by glycine with 500µM enhanced the expression of Pgc-1α in differentiated C2C12 cells.

### «Example 5»

In this example, a capsule-shaped mitochondrial function activator containing β-alanine and glycine was produced.

1.0 part by weight of β-alanine and 1.0 part by weight of glycine, and 0.5 part by weight of dextrin and mixed and homogenized. Then the mixtures were filled into hard capsules and to manufacture capsules with a content of 250 mg (β-alanine 100mg/capsule, glycine 100 mg/capsule).

### «Example 6»

In this example, a food composition for activating mitochondrial function in the form of a beverage containing β-alanine and glycine was produced.

35.5 g of sports drink powder, and 0.5g of β-alanine and 0.5g of glycine were added to water, to produce beverages with a total volume of 500mL.

### «Example 7»

In this example, a gel-shaped mitochondrial function activator including capsules containing β-alanine or glycine was produced.

7.1 parts by weight of sports drink powder, and 2 parts by weight of gelatin hydrolyzate, were added to an appropriate amount of water and dissolved by heating. Then, gel-like preparations of 150 g per serving was prepared by adding 0.1 part by weight of β-alanine, 0.1 part by weight of glycine to an appropriate amount of water, to make the total amount 100 parts by weight.

### «Example 8»

In this example, mitochondrial activation was examined by using a confocal laser scanning microscope.

The cells treated with β-alanine for 48 hours were prepared by the same procedure as in Example 1. C2C12 cells were seeded at a concentration of 3.0×10⁴ cells/mL or 5.0×10⁴ cells/mL, and Mito Tracker Green FM (Invitrogen) and Mito Tracker Red CMXRos (Invitrogen) were added simultaneously. The resulting cells were observed under a confocal laser scanning microscope (Olympus).

As shown in Fig. 8, the addition of 500 µM β-alanine strengthened the red fluorescence of Mito Tracker Red CMX Ros, and the addition of 1 mM β-alanine further strengthened the red fluorescence.

### «Example 9»

In this example, the fluorescence of Mito Tracker Red CMX Ros was measured using a flow cytometry.

The cells were prepared by repeating the procedure of Mito Tracker Red CMX Ros in Example 1 except that the treatment with β-alanine was set to 72 hours.

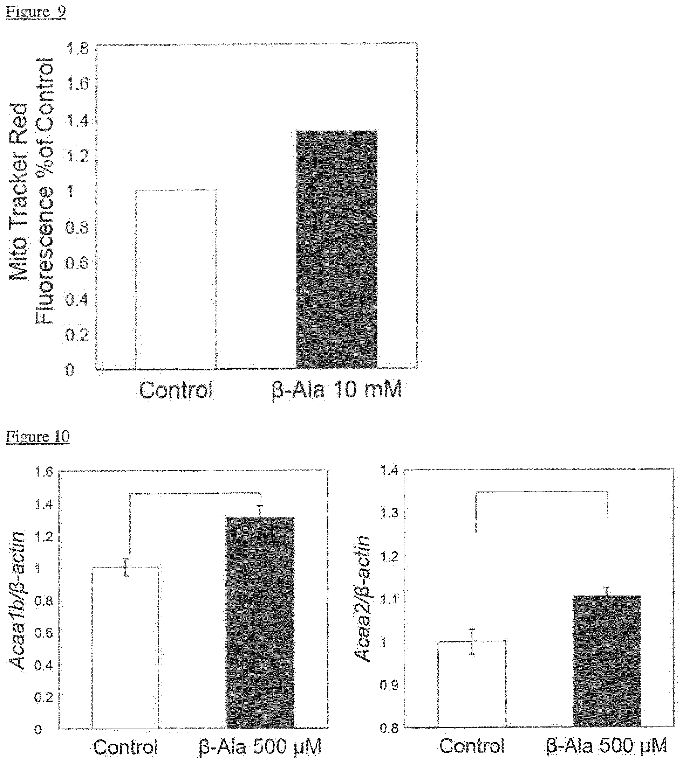

The resulting cells were analyzed by a flow cytometry (Beckman Coulter). As shown in Fig. 9, the addition of β-alanine increased the fluorescence of Mito Tracker Red. Therefore, mitochondria were activated by the addition of β-alanine.

### «Example 10»

In this example, the transcription of the genes for Acaalb and Acaa2 in β-alanine-treated mouse skeletal muscle-derived myoblast line, C2C12 cells was measured.

The procedure described in Example 4 was repeated except that the Acaalb forward primer and the Acaalb reverse primer, or the Acaa2 forward primer and the Acaa2 reverse primer were used instead of the Pgc-1α forward primer and the Pgc-1α reverse primer.
Acaalb forward: 5'-TGAGCGGTTTGGCGTTT-3' (SEQ ID NO: 5)
Acaalb reverse: 5'-CTTGTCACCCTTGTCATTCAGG-3' (SEQ ID NO: 6)
Acaa2 forward: 5'-TGCCCCTCAGTTCTTGTCTGT-3' (SEQ ID NO: 7)
Acaa2 reverse: 5'-AGGTGTGCGGTGATTCTGG-3' (SEQ ID NO: 8)

As shown in Fig. 10, the treatment with 500 µM of β-alanine enhanced the expression of Acaalb and Acaa2 in differentiated C2C12 cells.

### INDUSTRIAL APPLICABILITY

The mitochondrial function activator and the food composition for activating mitochondrial function of the present invention can be used for prevention or treatment of diseases caused by mitochondrial dysfunction, or for increasing slow muscle, enhancing

## Claims

1. A mitochondrial function activator comprising β-alanine or glycine, or a salt thereof.

2. The mitochondrial function activator according to claim 1, for improving fat burning, or aerobic exercise ability.

3. The mitochondrial function activator according to claim 1, for preventing or treating a disease caused by mitochondrial dysfunction.

4. The mitochondrial function activator according to claim 3, wherein the disease caused by mitochondrial dysfunction is a mitochondrial disease, neurodegenerative disease, immune neurological disease, cerebral ischemic disease, renal disease, muscle disease, or heart disease.

5. The mitochondrial function activator according to claim 4, wherein the mitochondrial disease is a Leigh encephalopathy, stroke-like episodes syndrome (MELAS), chronic progressive external ophthalmoplegia (CPEO), Kearns-Sayre syndrome (KSS), myoclonus epilepsy associated with ragged-red fibers (MERRF), Pearson syndrome, Leber's hereditary optic neuropathy (LHON), mitochondrial encephalomyopathy, Barth syndrome, or lactic acidosis.

6. The mitochondrial function activator according to claim 4, wherein the neurodegenerative disease is an amyotrophic lateral sclerosis (ALS), Parkinson's disease, Alzheimer's disease, Huntington's disease, Friedreich ataxia, multiple system atrophy, progressive supranuclear palsy, spinocerebellar degeneration, spinal muscular atrophy, spinal and bulbar muscular atrophy, or Charcot-Marie-Tooth disease.

7. The mitochondrial function activator according to claim 4, wherein the immune neurological disease is a Guillain-Barre syndrome, multiple sclerosis, Miller Fisher syndrome, chronic inflammatory demyelinating polyneuropathy, or myasthenia gravis.

8. The mitochondrial function activator according to claim 4, wherein the cerebral ischemic disease is a cerebral infraction.

9. The mitochondrial function activator according to claim 4, wherein the renal disease is a renal failure, amyloid kidney, membranous nephropathy, focal glomerular sclerosis, IgA nephropathy, acute tubular necrosis, nephrotic syndrome, diabetic nephropathy, gouty kidney, renal edema, kidney tumor, kidney ischemia disorder, renal ischemia-reperfusion disorder, or cystic kidney.

10. The mitochondrial function activator according to claim 4, wherein the muscle disease is a , progressive muscular dystrophy, myotonic dystrophy, congenital myopathy, metabolic myopathy, distal myopathy, inflammatory myopathy, age-related muscle atrophy (sarcopenia), or disuse muscular atrophy.

11. The mitochondrial function activator according to claim 4, wherein the heart disease is a myocardial infarction, cardiac failure, ischemic heart disease, or cardiac myopathy.

12. A food composition for activating mitochondrial function, comprising β-alanine or glycine, or a salt thereof.

13. A method for activating mitochondrial function, comprising a step of administering the mitochondrial function activator according to claim 1 to a subject.

14. The method for activating mitochondrial function according to claim 13, for improving a fat burning or improving an aerobic exercise ability of the subject.

15. An exercise training method for enhancing ability in aerobic exercise or increasing slow muscle, comprising a step of ingesting the mitochondrial function activator according to claim 1 to a subject.

16. A dietary method for enhancing ability in aerobic exercise or increasing slow muscle, comprising a step of ingesting the mitochondrial function activator according to claim 1 to a subject.

17. A Pgc-1α gene activator comprising β-alanine or glycine, or a salt thereof.

18. An Acaalb gene activator comprising β-alanine or glycine, or a salt thereof.

19. An Acaa2 gene activator comprising β-alanine or glycine, or a salt thereof.
